# FASCICULE DE BREVET EUROPEEN

(11) **EP 1 786 488 B1**
(45) Date de publication et mention de la délivrance du brevet: **16.04.2014**
(21) Numéro de dépôt: 05797766.2
(22) Date de dépôt: 04.08.2005
(51) Int. Cl.: A61M 1/02, A61M 1/34, A61M 1/00

(54) **DISPOSITIF D'AUTOTRANSFUSION A SEPARATION DE PHASES ET CONCENTRATION A POCHES AMOVIBLES**
AUTOTRANSFUSIONSVORRICHTUNG MIT PHASENTRENNUNG UND KONZENTRATION, UMFASSEND ENTFERNBARE BEUTEL
AUTOTRANSFUSION DEVICE WITH PHASE SEPARATION AND CONCENTRATION, COMPRISING REMOVABLE BAGS

(30) Priorité: 19.08.2004 FR 0451870
(43) Date de publication de la demande: 23.05.2007
(73) Titulaire: Direction et Priorités SA, 33170 Gradignan (FR)
(72) Inventeur: PEROVITCH, Philippe, F-33950 LEGE CAP FERRET (FR); GADRAT, Francis, F-33200 BORDEAUX (FR); CHASTENET, Bertrand, F-33320 EYSINES (FR)
(74) Mandataire: Fantin, Laurent
(86) Numéro de dépôt international: PCT/FR2005/050648
(87) Numéro de publication internationale: WO 2006/021728

(56) Documents cités:
- EP-A- 0 525 493
- DE-U1- 29 713 774
- US-A- 4 006 745
- US-A- 4 631 050
- US-A- 4 886 487
- US-A- 4 892 529

## Description

La présente invention concerne un dispositif d'autotransfusion à séparation des phases aqueuses et sanguines et à poches amovibles.

On connaît un dispositif d'autotransfusion à usage unique tout à fait performant ayant fait l'objet d'une demande de brevet français N° 2 600 537.

Un tel dispositif d'autotransfusion est particulièrement utile dans certaines circonstances dans lesquelles les méthodologies hospitalières connues sont inappropriées. En effet, de façon connue, en cas d'intervention chirurgicale délicate soit par la durée, soit par les risques de forte hémorragies, les praticiens prévoient des quantités de sang appropriées.

Cette pratique est admissible pour des interventions programmées, dans des lieux médicalement équipés et à proximité de banques du sang approvisionnées à cet effet.

On constate néanmoins que même dans ce cas, les coûts sont élevés, l'approvisionnement n'est pas toujours simple, surtout pour certains groupes sanguins plus spécifiques. De plus, une fois approvisionné, il est impossible de conserver les stocks non utilisés lors de l'intervention prévues avec des délais importants, actuellement au-delà de 35 jours et ceci impose dans ce cas des gestions de dates, ce qui engendre des contraintes administratives lourdes pour des centres médicaux dont ce n'est pas la vocation.

On sait aussi que dans le cas des transfusions sanguines, dans certains pays, il est difficile d'avoir des certitudes quant à la qualité du sang et de son épuration, ce qui fait courir des risques au patient transfusé. Notamment, il peut y avoir des contaminations potentielles par le virus du HIV, des hépatites, de la tuberculose ou de la syphilis mais aussi par des agents pathogènes indétectés ou indétectables comme les agents pathogènes non-conventionnels.

De plus, lors d'une transfusion, il est plus judicieux de pouvoir au moins récupérer et retransfuser une partie du sang propre à l'individu, avec ses anticorps et toutes les autres molécules qui lui sont propres que de lui transfuser du sang d'une banque du sang.

Il existe d'autres circonstances, notamment lors des catastrophes naturelles, lors des guerres, dans lesquelles l'approvisionnement en sang est impossible compte tenu des quantités mises en jeu. L'autotransfusion reste la seule solution. Certaines populations n'acceptent pas, pour des raisons religieuses, par conviction ou pour toute autre raison de recevoir un sang transfusionnel provenant d'une tierce personne, l'autotransfusion est là encore le seul moyen de sauver des vies.

Il est aussi nécessaire de rappeler que, dans le cas des accidents, les délais d'intervention peuvent s'avérer très réduits et l'approvisionnement à partir d'une banque du sang est très difficile voire impossible dans des laps de temps aussi brefs, en particulier dans les pays qui ne disposent pas des organisations ou structures pour la collecte, la collection, le contrôle et la mise en place de transfusion sanguine. Dans ces cas, l'autotransfusion par récupération du sang hémorragique représente une solution immédiatement disponible.

Il convient ensuite de distinguer deux applications particulières la première étant per opératoire et la seconde étant post opératoire, certaines interventions pouvant être concernées par les deux applications avec une pratique d'une autotransfusion au cours de l'intervention et sa poursuite après l' intervention, par récupération du sang hémorragique au moyen d'un drain externe, généralement dans les 6 heures qui suivent l'intervention.
- Dans le cas de l'autotransfusion per opératoire, il convient de pouvoir retransfuser le sang recueilli directement chez le patient, presque en continu. Or, lors du recueil, ceci de façon connue, ce sang doit nécessairement être dilué et additionné d'agents anti-coagulants pour préserver sa qualité transfusionnelle.

Ces actions apparaissent nécessaires car en utilisant un liquide vecteur du sang hémorragique recueilli, les globules rouges peuvent être ainsi protégés des traumatismes physiques directs lors du contact mécanique avec les filtres et autres tubulures. Cette dilution dans un liquide vecteur diminue également le contact des globules rouges avec l'air, limitant ainsi fortement leur hémolyse. Ensuite, le sang récupéré doit être retransfusé au patient mais on se heurte à des problèmes importants.

En effet, dans le cas de transfusion de volumes de sang trop dilués, on peut provoquer des phénomènes d' hypervolémie par ces volumes liquidiens transfusés trop importants et des syndromes d'hypo-coagulabilité par un volume transfusé d'anti-coagulants trop important.

Afin d'éviter cela, il est nécessaire de recourir à l'usage de machines très complexes et très chères pour extraire les liquides d'hémodilution, dites machines de lavage/centrifugation.

De plus, lors de l'autotransfusion du sang directement prélevé et seulement anticoagulé et hémodilué, il est possible de retransfuser des substances biologiques activées ou dégradées et susceptibles de provoquer des effets secondaires. On peut trouver des histamines, des kallicréines ou des kinines, des facteurs plasmatiques plus ou moins dégradés dont il vaut mieux se débarrasser ou encore des petites protéines issues des traumatismes cellulaire.

Pour les conditions d'autotransfusion *per* opératoire, le procédé selon l'invention consiste donc à recueillir le sang, à le diluer et à l'anti-coaguler simultanément au prélèvement et proportionnellement à la quantité prélevée, puis à filtrer et à séparer la phase liquidienne de la phase contenant les globules rouges permettant ainsi d'une part une concentration de la phase recueillie et destinée à être retransfusée et d'autre part une collecte de la phase liquidienne à éliminer.
- Dans le cas d'une intervention *post* opératoire, le sang est recueilli directement par un drain et le liquide écoulé ne subit aucun traitement de dilution ou d'anti-coagulation puisqu'il est dans ce cas, naturellement prêt à être retransfusé, à condition qu'il soit recueilli dans des conditions stériles bien entendu.

On connaît le brevet US 4 886 487qui décrit un appareil d'autotransfusion avec un circuit du sang muni d'un étage de dilution et de recueil du sang et un étage de concentration et de réinjection du sang au patient. Ce dispositif comprend une succession d'éléments technique sans présenter un dispositif monolithique avec une commande multifonctions. Ce dispositif comprend une pompe avec une circulation complexe.

On connaît également le brevet US 4 006 745 qui décrit un dispositif de traitement du liquide hémorragique d'un patient avec une base et une tête ainsi qu'une valve de liaison amovible mais aucune commande unique n'est prévue.

La présente invention propose un dispositif, à usage unique, qui permet une mise en oeuvre dans chacune des conditions *per* ou *post* opératoire, indépendamment mais aussi dans le cas d'une intervention nécessitant une poursuite de l'autotransfusion depuis la phase *per* opératoire jusqu'à la phase *post* opératoire, ceci en utilisant le même dispositif.

Le procédé et le dispositif associé sont maintenant décrits en détail selon un mode de réalisation particulier, non limitatif, à partir des dessins annexés, dessins dont les différentes figures représentent :
- figure 1, une vue du dispositif complet équipé de son étage *per* opératoire,
- figure 2, une vue détaillée en perspective des moyens de séparation/concentration et des moyens de récupération des globules rouges d'une part et des liquides d'hémodilution et d'anti-coagulation, d'autre part,
- figure 3, une vue de l'étage post opératoire du dispositif.

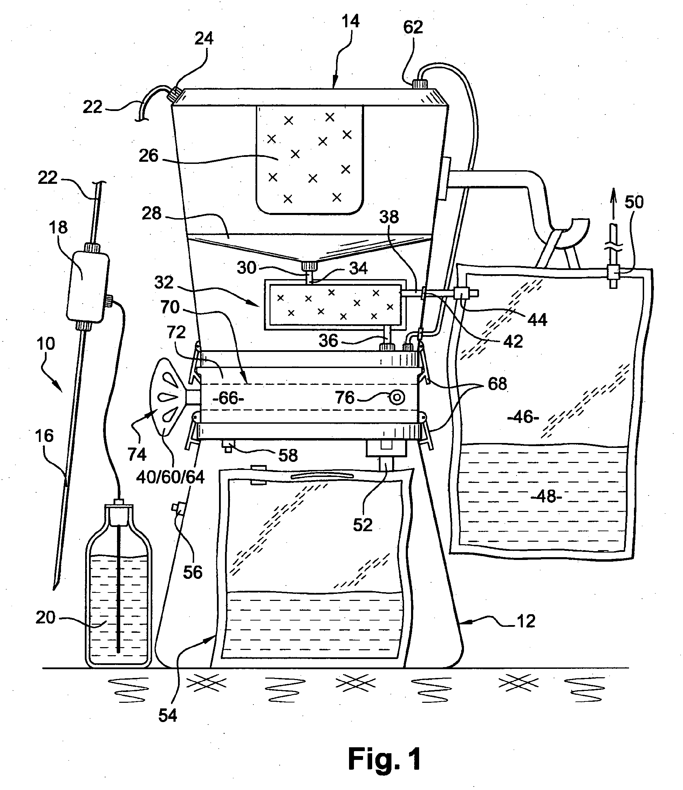

Sur la figure 1, on a représenté des moyens 10 de prélèvement du sang d'un patient au cours d'une intervention, une base 12 du dispositif selon l'invention et une tête 14 amovible du dispositif pour une application per opératoire selon l'invention.

Les moyens 10 de prélèvement sont connus en eux-mêmes et comprennent une canule 16 d'aspiration du sang hémorragique, des moyens 18 de dosage intégré d'agents 20 diluants et anti-coagulants et un tube 22 d'évacuation.

Les moyens 18 de dosage permettent d'adjoindre en continu et proportionnellement au volume de sang recueilli, la quantité nécessaire d'agents 20 diluants et anti-coagulants.

Le tube 22 d'évacuation est relié à un piquage 24 ménagé en partie supérieure de la tête 14 amovible.

Ce piquage se prolonge vers un filtre 26, par exemple avec une maille de 40*µ*m pour donner une idée.

Ce filtre 26 est rapporté sur la partie haute de la tête 14.

Ce filtre est disposé au-dessus d'un collecteur 28 en forme d'entonnoir et muni d'une sortie 30.

Un filtre 32 de séparation /concentration est rapporté sur cette sortie 30 par son tube 34 d'admission.

Ce filtre 32 de séparation/concentration est muni d'une première sortie 36 et d'une seconde sortie 38. La membrane de ce filtre, réalisée à partir d'un polymère ou de cellulose est hydrophile et présente une porosité de l'ordre de 0,5 à 5*µ*m.

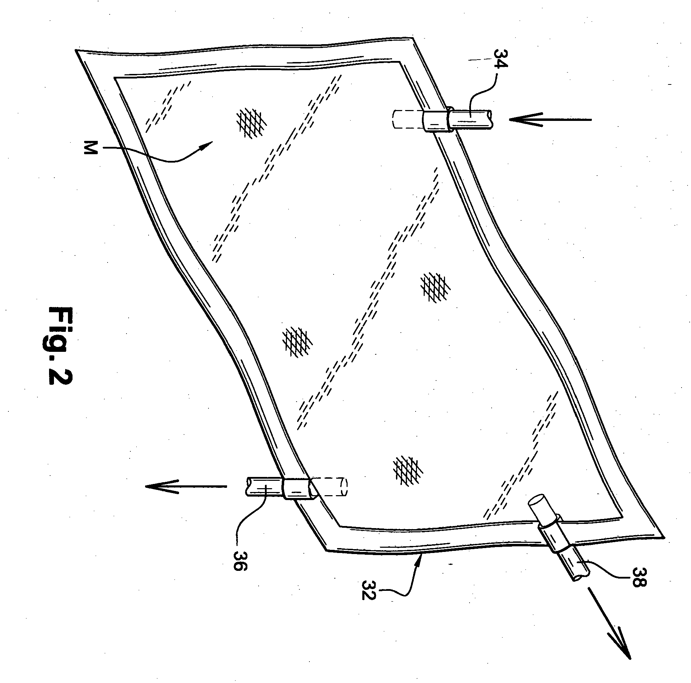

On se reporte utilement à la figure 2. Le filtre de séparation/concentration est du type poche souple dans laquelle est interposée une membrane M qui sépare la poche souple en deux volumes V1 et V2. Cette membrane est apte à retenir les globules rouges dans le volume V1, du côté admission et apte à laisser passer les autres liquides à travers sa paroi vers V2.

Aussi la première sortie 36 est connectée au volume V1, en partie inférieure, permettant l'évacuation des globules rouges concentrés pour représenter entre 50 et 60% du volume associé à une fraction des liquides, pour donner un ordre d'idée. La première sortie 36 comprend des moyens 40 d'ouverture et de fermeture.

La seconde sortie 38 est connectée au volume V2.

Cette seconde sortie est rapportée sur un piquage 42 traversant. Ce piquage 42 est relié à un piquage 44 porté par des moyens 46 de recueil des liquides 48, en l'occurrence une poche souple. Cette poche souple est d'un grand volume, même si pour des raisons pratiques, celle-ci est réduite sur les dessins. Elle doit pouvoir contenir tous les excédents des liquides d'hémodilution récupérés à travers le filtre 32, lors d'une intervention, sans nécessiter de changement.

Ces moyens 46 de recueil des liquides sont munis d'un piquage 50 d'aspiration sur lequel il peut être connecté une source de vide.

La première sortie 36 est reliée à un piquage 52 d'admission de moyens 54 de recueil de la fraction sanguine concentrée. Ces moyens 54 sont en l'occurrence une poche souple. Le piquage 52 d'admission est muni d'une connexion permettant un retrait de la poche et une obturation simultanée de ladite poche afin de prévenir toute fuite de sang et/ou pour interdire toute mise à l'air libre de la fraction sanguine concentrée contenu dans les moyens 54 de recueil.

Une telle poche, selon l'invention, comprend un bouchon portant deux pas de vis inversés en sorte de visser une partie du bouchon et d'obturer la poche tandis que cette rotation du bouchon provoque simultanément la déconnexion du piquage sur lequel il est rapporté initialement par vissage. La poche reste immobile en rotation pendant cette manoeuvre.

Cette base 12 comprend en outre un piquage 56 avec obturateur pour permettre de casser le vide à l'intérieur de cette base.

Il est également prévu dans cette base un piquage 58 permettant la mise sous vide du volume intérieur de cette base. Ce piquage est commandé par des moyens 60 d'ouverture/fermeture relié à une source de vide.

Il est également prévu dans la tête 14 un ensemble de mise sous vide du volume intérieur de la tête 14 comprenant un piquage 62 en partie supérieure relié à des moyens d'ouverture/fermeture 64 relié à une source de vide.

La source de vide commune est reliée à une interface 66 interposée entre la base 12 et la tête 14. Cette interface est solidaire de façon étanche de la base 12 et de la tête 14. Cette interface 66 possède un joint d'étanchéité avec la base 12 et un joint d'étanchéité avec la tête 14 ainsi que des moyens 68 de liaison mécanique et amovible avec ladite base et avec ladite tête.

Cette interface est munie d'une commande 70 unique multifonctions, permettant de regrouper les moyens d'ouverture/fermeture 40, 60 et 64. Une telle commande est constituée, dans le mode de réalisation présenté, d'un conduit 72 muni d'un piston équipé de piquages, de passages internes et de joints adaptés, non figurés mais à la portée de l'homme de l'art ayant connaissance des fonctionnalités à remplir et détaillées ci-avant. Ce piston est manoeuvrable de l'extérieur du dispositif par une molette 74 portant les indications et repères nécessaires. Il est aussi relié par un piquage 76 à une source de vide générale qui peut être identique à celle utilisée pour les autres besoins.

Le fonctionnement du dispositif tel qu'il vient d'être décrit, pendant une intervention opératoire, est maintenant décrit.

Le dispositif est connecté à une source de vide par le piquage 76. Une poche 54 est disposée dans la base 12. Cette poche est stérile et connectée de façon étanche permettant une déconnexion ultérieure avec une obturation stérile automatique.

Parallèlement, une poche 46 souple est également rapportée sur le piquage 44, ceci sans nécessité d'un environnement stérile, les liquides recueillis étant destinés à être détruits. Néanmoins, cette poche peut aussi être équipée pour des raisons pratiques de manipulation, d'asepsie d'un bouchon identique à celui de la poche 54 bouchon qui permet d'obturer la poche et de provoquer simultanément la déconnexion du piquage sur lequel il est rapporté initialement par vissage.

Le piquage 56 est fermé. La molette 74 est manoeuvrée dans une position permettant la mise sous vide de la base 12 et de la tête 14 par le piquage 62.

La poche 46 est également reliée à une source de vide par son piquage 50. Cet ensemble est symbolisé théoriquement, simplifié, de la sorte mais dans la réalité, comme il s'agit d'une poche souple, pour permettre son remplissage, il convient de disposer la poche dans un réceptacle qui est, lui, mis en dépression, de façon analogue à la base 12. Ceci permet d'écarter les parois de la poche et de générer un vide dans la poche assurant ainsi le remplissage et non un placage des parois de ladite poche.

Le praticien procède à l'aspiration des liquides épanchés du patient grâce à la canule 16.

Simultanément, un ou des anti-coagulants et un ou plusieurs diluants permettent de traiter les liquides et d'éviter la dégradation des globules rouges.

Sous l'effet de la dépression, les liquides sont aspirés par la tête 14 et passent à travers le piquage 24, et à travers le filtre 26 permettant de retenir les particules de plus grandes tailles, au-delà de 40*µ*m.

Le filtrat, aspiré par la dépression dans la tête 14, est recueilli dans le collecteur 28 puis passe dans le filtre 32 de séparation/concentration.

Les globules rouges et une fraction des liquides sont retenus par la membrane de ce filtre et sont dirigés, du fait de la dépression générée dans la base 12 vers la poche 54.

Le filtrat 48, issu du filtre 32 de séparation/concentration, de porosité moyenne inférieure à 5*µ*m, est aspiré du fait de la dépression de la poche 46 vers cette poche. Ce filtrat 48 est exempt de globules rouges.

Lorsque la poche 54 contenant le sang hémodilué et concentré est pleine, la molette, actionnée de façon adaptée, permet le maintien du vide dans la tête 14 tout en interrompant le vide dans la base 12.

La dépression est également maintenue dans la poche des moyens 46 de recueil. Le vide dans la base est cassé par manoeuvre du piquage 56.

Les moyens d'assujettissement mécanique de la tête 14 sur la partie supérieure de l'interface 66 d'une part et de la base 12 sur la partie inférieure de cette interface 66, permettent de désolidariser ladite tête et ladite base.

La manoeuvre de la connexion 52 de la poche des moyens 54 de recueil, permet de fermer cette première poche et de la retirer tandis qu'une autre poche est immédiatement mise en place si nécessaire.

La tête est de nouveau assujettie sur la base 12 et la manoeuvre de la molette 74 permet de remettre en dépression la base 12 après avoir manoeuvré le piquage 56 de façon adaptée, vers la position fermeture.

Le collecteur 28 assure une fonction tampon pendant cette opération de changement de poche, sans qu'il se produise une quelconque interruption pour le praticien.

La poche contenant le sang concentré peut être conservée ou immédiatement utilisée pour une transfusion du sang ainsi recueilli vers le patient durant l'intervention, le sang étant parfaitement apte à cette opération de transfusion. On note que ce sang est concentré et évite les effets secondaires des procédés de l'art antérieur.

De plus, ce sang concentré apporte un capital cellulaire accroissant la capacité d'oxygénation des tissus de l'organisme du patient.

La présente invention prévoit aussi une tête permettant le drainage et la récupération du sang hémorragique en phase post opératoire.

Les mêmes références sont conservées pour les éléments communs et les parties nouvelles sont référencées en ajoutant 100.

Dans le cas post opératoire, le praticien a disposé un drain permettant le recueil du sang hémorragique qui est de très bonne qualité. Ce sang hémorragique, qui n'est pas sujet à coagulation, ne nécessite pas de traitement spécifique si ce n'est un traitement de filtration à travers un filtre de porosité moyenne inférieure à 40 *µ*m. On sait que les volumes recueillis peuvent varier entre 0,5 et 2 litres, ce qui est particulièrement important pour un patient après une intervention.

Ce sang ainsi recueilli, pour autant que ce soit dans des conditions adaptées d'asepsie, peut être retransfusé directement au patient.

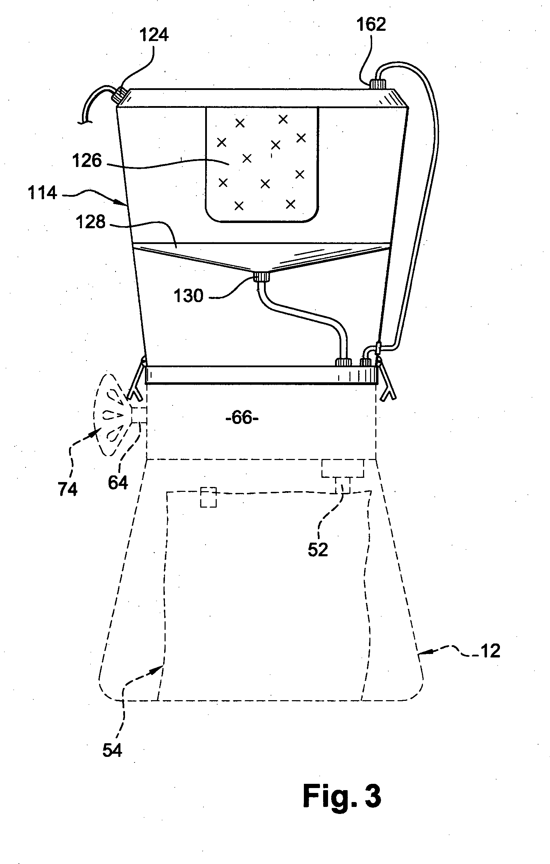

Dans ce cas, le dispositif représenté sur la figure 3 comprend une base 12 identique , une interface 66 identique et une tête 114 différente.

Cette tête 114 comprend uniquement un filtre 126 à maille de 40 *µ*m environ.

Le piquage 162 pour une mise sous vide de la tête 114 est conservé, toujours relié par un piquage 64 à des moyens d'ouverture et de fermeture par la commande unique multifonctions.

Sous le filtre, un collecteur 128 est également prévu qui débouche par son piquage 130 directement à travers l'interface 66 dans la base 12 par un piquage 52 dont l'ouverture et la fermeture sont également commandées par la commande unique multifonctions.

Le filtre de séparation/concentration est supprimé.

Le piquage 52 reçoit comme précédemment des moyens de recueil 54, en l'occurrence une poche souple.

Le piquage 42 est également supprimé sur la tête puisqu'il n'y a plus de moyens de collecte de liquides : aucune adjonction n'ayant été effectuée, il n'y a aucune concentration à réaliser.

La tête 114 est bien entendu munie d'un piquage 124 pour permettre la connexion avec le drain.

La poche des moyens 54 de recueil peut être retirée pour un retransfusion de son contenu comme précédemment.

La tête 114 est maintenue en dépression pour recueillir les écoulements venus du drain tandis que le vide est cassé par manoeuvre au niveau du piquage 56 dans la base après avoir manoeuvré la molette 74 pour interrompre la mise en dépression de la base 12.

La poche est retirée et fermée simultanément.

Une nouvelle poche est mise en place et le piquage 56 étant refermé, la nouvelle mise en dépression de la base provoque le remplissage de la nouvelle poche.

On note comme précédemment que le collecteur et une partie du volume de la tête permettent d'assurer une fonction tampon et d'assurer le recueil en continu des écoulements du drain pendant le changement de poche dans la base 12.

Le délai de recueil de ce sang hémorragique ne peut excéder une durée de 6 heures après l'intervention.

On peut ainsi récapituler les nombreux avantages de ce dispositif.

On constate que l'architecture générale permet de standardiser les différents modules à savoir, base 12 et interface 66 qui sont communs et les deux têtes 14 per opératoire et 114 post opératoire.

A l'aide de ces quatre modules, il est possible de répondre à de nombreuses situations.

Dans la phase per opératoire, le dispositif permet une mise en oeuvre nouvelle qui consiste à traiter le sang en continu pour permettre sa retransfusion immédiate chez le même patient.

Sur le plan médical, un tel dispositif évite les syndromes d'hypo-coagulabilité par un volume transfusé d'anti-coagulants trop important, il réduit aussi la retransfusion de substances biologiques activées ou dégradées et susceptibles de provoquer des effets secondaires. De plus le sang retransfusé après concentration contribue à l'apport d'un capital cellulaire accroissant la capacité d'oxygénation des tissus de l'organisme du patient.

Le dispositif autorise la récupération des poches de recueil du sang ou des écoulements du drain dans les meilleures conditions, au fur et à mesure de leur remplissage pour une utilisation immédiate, ceci sans jamais interrompre le fonctionnement du dispositif.

La collecte des liquides résiduaires provenant de la concentration dans la phase per opératoire est également effectuée directement dans des contenants du type poche souple sans manipulation, sans vérification, sans souillure de quelque sorte que ce soit.

Le dispositif reste compact malgré les nombreuses fonctionnalités, ce qui est un atout en milieu médical.

La manipulation à l'aide d'une seule molette multifonctions est extrêmement simple et évite toute erreur de manipulation comme cela peut se produire lorsque plusieurs organes doivent être manoeuvrés dans un certain ordre qui plus est, alors qu'il y a d'autres actes à accomplir plus importants simultanément. Ceci supprime donc le stress lié à l'utilisation d'un tel dispositif pour laisser toute la concentration des personnels focalisés sur les actes simultanés ou complémentaires.

Un tel dispositif peut encore être perfectionné par intégration d'un filtre supplémentaire à déleucocyter le sang issu du premier filtre à 40*µ*m. Dans ce cas, un tel filtre à déleucocyter est interposé sur la sortie 36 du filtre 32 de séparation/concentration dans le cas de la tête per opératoire et/ou sur la sortie 130 dans le cas de la tête 114 post opératoire.

La poche de récupération dans le cas de la présente invention peut en plus de posséder un piquage avec des moyens d'obturation simultanée au retrait, comporter une sortie avec une obturation destinée à être perforée lors de l'utilisation.

## Revendications

1. Dispositif de traitement du liquide hémorragique d'un patient lors d'une intervention chirurgicale ou postérieurement, comprenant une base (12), au moins une tête (14, 114) et une interface de liaison (66), ladite base (12) comprenant des moyens (54) de recueil de façon stérile d'au moins une partie du liquide issu de la tête (14, 114), ladite tête (14, 114) comprenant des moyens (26, 126, 32) de traitement du liquide hémorragique, chaque tête étant fixée de façon amovible sur l'ensemble interface (66) de liaison et base (12), en sorte de permettre l'accès auxdits moyens (54) de recueil afin d'en effectuer le changement, **caractérisé en ce que** l'interface de liaison comprend une commande unique multifonctions (70) permettant de regrouper des moyens d'ouverture/fermeture (40) de la base (12), des moyens (60) d'ouverture/fermeture de la base (12) reliés à une source de vide, et des moyens (64) d'ouverture/fermeture de ladite au moins une tête (14, 114) reliés à une source de vide.

2. Dispositif de traitement du liquide hémorragique selon la revendication 1, **caractérisé en ce qu'**il comprend une tête (14) *per* opératoire et une tête (114) *post* opératoire, chacune apte à être rapportée sur un même ensemble interface (66) et base (12).

3. Dispositif de traitement du liquide hémorragique selon la revendication 2, **caractérisé en ce que** les moyens de traitement du liquide hémorragique de la tête (14) *per* opératoire comprennent des moyens (26) de filtration et des moyens (32) de séparation/concentration munis d'une première sortie (36) permettant l'évacuation des globules rouges et d'une fraction des liquides et d'une seconde sortie (38) reliée à des moyens (46) de recueil des liquides (48) exempts de globules rouges.

4. Dispositif de traitement du liquide hémorragique selon la revendication 2 ou 3, **caractérisé en ce que** les moyens (32) de séparation/concentration comprennent une poche souple, une membrane M de séparation en deux volumes V1 et V2 de cette poche souple, ladite membrane M étant apte à retenir les globules rouges dans le volume V1, du côté admission au droit d'un piquage (34) et apte à laisser passer les autres liquides à travers sa paroi vers le volume V2 lui-même relié par des piquages (42,44) aux moyens (46) de recueil des liquides (48) exempts de globules rouges.

5. Dispositif de traitement du liquide hémorragique selon la revendication 3 ou 4, **caractérisé en ce que** les moyens de recueil des liquides (48) exempts de globules rouges est une poche munie de moyens (50) de mise sous vide.

6. Dispositif de traitement du liquide hémorragique selon l'une quelconque des revendications précédentes 1 à 5, **caractérisé en ce que** les moyens (54) de recueil de façon stérile d'au moins une partie du liquide issu de la tête (14) comprennent une poche souple rapportée de façon amovible sur un piquage (52) d'admission muni d'une connexion permettant un retrait de ladite poche et une obturation simultanée pour interdire toute fuite de sang et/ou toute mise à l'air libre de son contenu.

7. Dispositif de traitement du liquide hémorragique selon la revendication 6, **caractérisé en ce que** la connexion permettant un retrait de ladite poche et une obturation simultanée pour interdire toute fuite de sang et/ou toute mise à l'air libre de son contenu comprend un bouchon portant deux pas de vis inversés en sorte de visser une partie du bouchon et d'obturer la poche de rotation du bouchon et de provoquer simultanément la déconnexion du piquage sur lequel il est rapporté initialement par vissage.

8. Dispositif de traitement du liquide hémorragique selon l'une quelconque des revendications précédentes 1 à 7, **caractérisé en ce qu'**il comprend un filtre à déleucocyter disposé dans la tête (14) *per* opératoire et/ou dans la tête (114) post opératoire.

## Patentansprüche

1. Vorrichtung zur Behandlung von hämorrhagischer Flüssigkeit eines Patienten während einer chirurgischen Intervention oder danach mit einer Basis (12), mit wenigstens einen Kopf (14, 114) und mit einer Verbindungsschnittstelle (66), wobei die Basis (12) Mittel (54) zur sterilen Aufnahme wenigstens eines Teils der vom Kopf (14, 114) abgegebenen Flüssigkeit aufweist und der Kopf (14, 114) Mittel (26, 126, 32) zur Behandlung der hämorrhagischen Flüssigkeit aufweist, wobei jeder Kopf auf lösbare Weise auf der von der Verbindungsschnittstelle (66) und der Basis (12) gebildeten Einheit derart angebracht ist, dass ein Zugang zu den genannten Aufnahmemitteln (54) für deren Auswechslung gewährt ist, **dadurch gekennzeichnet, dass** die Verbindungsschnittstelle eine einzige multifunktionale Steuerung (70) aufweist, die es gestattet, Mittel zur Öffnung/Schließung (40) der Basis (12), Mittel (60) zur Öffnung/Schließung der Basis (12), die mit einer Vakuumquelle verbunden sind, und mit einer Vakuumquelle verbundene Mittel (64) zur Öffnung/Schließung des wenigstens einen Kopfes (14, 114) umzustellen.

2. Vorrichtung zur Behandlung von hämorrhagischer Flüssigkeit nach Anspruch 1, **dadurch gekennzeichnet, dass** diese einen Kopf (14) per Operation und einen Kopf (114) *post* Operation aufweist, die beide dazu eingerichtet sind, auf der gleichen, von Schnittstelle (66) und Basis (12) gebildeten Einheit angebracht zu werden.

3. Vorrichtung zur Behandlung von hämorrhagischer Flüssigkeit nach Anspruch 2, **dadurch gekennzeichnet, dass** die Behandlungsmittel der hämorrhagischen Flüssikeit des Kopfes (14) per Operation Mittel (26) zur Filtrierung und Mittel (32) zur Trennung/Konzentration aufweisen, die mit einem ersten Ausgang (36) versehen sind, der die Entleerung der roten Blutkörperchen und eines Teils der Flüssigkeiten gestattet, und die mit einem zweiten Ausgang (38) versehen sind, der mit Mitteln (46) zur Aufnahme der Flüssigkeiten (48) ohne rote Blutkörperchen verbunden ist.

4. Vorrichtung zur Behandlung von hämorrhager Flüssigkeit nach Anspruch 2 oder 3, **dadurch gekennzeichnet, dass** die Mittel (32) zur Trennung/Konzentration eine weiche Tasche aufweisen, eine Membran M, um diese weiche Tasche in zwei Volumen V1 und V2 zu trennen, wobei die Membran M dazu eingerichtet ist, die roten Blutkörperchen in Volumen V1 zurückzuhalten, sich einlassseitig im Bereich eines Anschlusses (34) befindet und dazu eingerichtet ist, die anderen Flüssigkeiten durch seine Wand zum Volumen V2 hindurchzulassen, das durch Anschlüsse (42, 44) mit den Mitteln (46) zur Aufnahme von Flüssigkeiten (48) ohne rote Blutkörperchen verbunden ist.

5. Verfahren zur Behandlung von hämorrhager Flüssigkeit nach Anspruch 3 oder 4, **dadurch gekennzeichnet, dass** die Mittel zur Aufnahme von Flüssigkeiten (48) ohne rote Blutkörperchen eine Tasche sind, die mit Mitteln (50) zur Vakuumerzeugung versehen ist.

6. Vorrichtung zur Behandlung von hämorrhager Flüssigkeit nach einem der vorhergehenden Ansprüche 1 bis 5, **dadurch gekennzeichnet, dass** die Mittel (54) zur sterilen Aufnahme wenigstens eines Teils der vom Kopf (14) abgegebenen Flüssigkeit eine weiche Tasche aufweisen, die auf lösbare Weise an einer Zuführung (52) angebracht ist, die mit einer Verbindung versehen ist, die einen gleichzeitigen Abzug und Verschluss der Tasche gestattet, um zu verhindern, dass Blut verloren geht und/oder deren Inhalt Umgebungsluft ausgesetzt ist.

7. Verfahren zur Behandlung von hämorrhager Flüssigkeit nach Anspruch 6, **dadurch gekennzeichnet, dass** die Verbindung, die einen gleichzeitigen Abzug und Verschluss gestattet, um zu verhindern, dass Blut verloren geht und/oder deren Inhalt Umgebungsluft ausgesetzt ist, einen Verschluss mit gegenläufigen Gewinden aufweist, um einen Teil des Verschlusses zu schrauben und die Tasche durch die Drehung des Verschlusses zu verschließen und gleichzeitig die Trennung vom Anschluss zu bewirken, an dem dieser ursprünglich durch Schrauben angebracht worden ist.

8. Vorrichtung zur Behandlung von hämorrhager Flüssigkeit nach einem der vorhergehenden Ansprüche 1 bis 7, **dadurch gekennzeichnet, dass** diese einen Filter zum Entleukozytieren aufweist, der im Kopf (14) per Operation und/oder im Kopf (114) *post* Operation angeordnet ist.

## Claims

1. A device for treating haemorrhagic liquid in a patient during a surgical operation or subsequently, comprising a base (12), at least one head (14, 114) and a connecting interface (66), said base (12) comprising means (54) for the sterile collection of at least some of the liquid issuing from the head (14, 114), said head (14, 114) comprising means (26, 126, 32) for treating the haemorrhagic liquid, each head being removably fixed to the connecting interface (66) and base (12) assembly, so as to allow access to said collection means (54) in order to change them, **characterised in that** the connecting interface comprises a unique multifunction control (70) grouping together means (40) for opening/closing the base (12), means (60) for opening/closing the base (12) connected to a vacuum source, and means (64) for opening/closing said at least one head (14, 114) connected to a vacuum source.

2. A device for treating haemorrhagic liquid according to claim 1, **characterised in that** it comprises a peroperative head (14) and a postoperative head (114), each able to be attached to the same interface (66) and base (12) assembly.

3. A device for treating haemorrhagic liquid according to claim 2, **characterised in that** the means for treating haemorrhagic liquid of the peroperative head (14) comprises filtration means (26) and separation/concentration means (32) provided with a first outlet (36) for discharging red corpuscles and a fraction of the liquids and a second outlet (38) connected to means (46) for collecting liquids (48) free from red corpuscles.

4. A device for treating haemorrhagic liquid according to claim 2 or 3, **characterised in that** the separation/concentration means (32) comprises a flexible pouch, a membrane M for separating this flexible pouch into two volumes V1 and V2, said membrane M being able to retain the red corpuscles in the volume V1, on the inlet side in line with a tapping (34), and able to allow the other liquids to pass through its wall to the volume V2 itself connected by tappings (42, 44) to the means (46) for collecting liquids (48) free from red corpuscles.

5. A device for treating haemorrhagic liquid according to claim 3 or 4, **characterised in that** the means for collecting liquids (48) free from red corpuscles is a pouch provided with means (50) for creating a vacuum.

6. A device for treating haemorrhagic liquid according to any one of the preceding claims 1 to 5, **characterised in that** the means (54) for collecting, in a sterile fashion, at least some of the liquid issuing from the head (14) comprises a flexible pouch removably attached to an inlet tapping (52) provided with a connection allowing removal of said pouch and simultaneous closure in order to prevent any leakage of blood and/or any exposure of its content to the open air.

7. A device for treating haemorrhagic liquid according to claim 6, **characterised in that** the connection allowing removal of said pouch and simultaneous closure to prevent any leakage of blood and/or any exposure of its content to open air comprises a stopper carrying two reversed screw threads so as to screw part of the stopper and to close off the pouch of rotation of the stopper and to simultaneously cause the disconnection of the tapping to which it is attached initially by screwing.

8. A device for treating haemorrhagic liquid according to any one of the preceding claims 1 to 7, **characterised in that** it comprises a leucocyte-removal filter disposed in the peroperative head (14) and/or in the postoperative head (114).
